# EUROPEAN PATENT SPECIFICATION

(11) **EP 1 157 969 B1**
(45) Date of publication and mention of the grant of the patent: **30.08.2006**
(21) Application number: 01304333.6
(22) Date of filing: 16.05.2001
(51) Int. Cl.: C01C 1/12, C07C 253/34

(54) **Process for recovery and recycle of ammonia from a reactor effluent stream**
Verfahren zur Wiedergewinnung und Wiederverwendung von Ammoniak aus einem Reaktoreffluentstrom
Procédé pour récuperation et recyclage d'ammoniac d'un effluent d'un reacteur

(30) Priority: 23.05.2000 US 206365 P; 03.05.2001 US 847345
(43) Date of publication of application: 28.11.2001
(60) Divisional application: 06075922.2
(73) Proprietor: ROHM AND HAAS COMPANY, Philadelphia, Pennsylvania 19106-2399 (US)
(72) Inventor: Benderly, Abraham, Houston, Texas 77906 (US); Decourcy, Michael Stanley, Houston, Texas 77062 (US); Myers, Ronald Eugene, Houston, Texas 77062 (US)
(74) Representative: Kent, Venetia Katherine

(56) References cited:
- US-A- 3 718 731
- US-A- 3 985 863
- US-A- 4 287 162
- US-A- 5 895 635

## Description

The present invention relates to an improved process for the recovery and regeneration of ammonia, e.g., ammonia contained in the effluent obtained from a reaction zone where ammonia and oxygen are reacted with a paraffin to produce the corresponding aliphatic nitrile. In particular, the present invention relates to minimization of ammonium carbamate formation, and minimization of contamination in downstream processes resulting from the presence of ammonium carbamate such as, for example, in a process for the recovery and regeneration of unreacted ammonia contained in the effluent passing from a reaction zone wherein ammonia and oxygen are reacted with propane to produce acrylonitrile, or isobutane to produce methacrylonitrile.

U.S. Pat. Nos. 3,936,360 and 3,649,179 are each directed to a process for the manufacture of acrylonitrile utilizing propylene, oxygen and ammonia as the reactants. These gases are passed over a catalyst in a fluid bed reactor to produce acrylonitrile which passes from the reactor to a recovery and purification section. This reaction also has some unreacted ammonia which is typically removed from the process by treatment in the quench column with an acid. The '179 patent discloses that the quench acid may be either sulfuric, hydrochloric, phosphoric or nitric acid. The '360 patent discloses the use of sulfuric acid in the quench to remove the unreacted ammonia. In the manufacture of acrylonitrile using propylene as the hydrocarbon source, the preferred embodiments clearly utilize sulfuric acid with the resulting formation of ammonium sulfate. Typically, the ammonium sulfate is either recovered and sold as a co-product (fertilizer) or combined with other heavy organics produced in the process and deep-welled for environmentally safe disposal.

British Patent 222,587 is directed to ammonia recovery from an ammonia-containing gas mixture utilizing an aqueous phosphoric acid solution, an aqueous solution of ammonium hydrogen phosphate , or mixtures thereof. The ammonia is recovered by heat decomposition and dissolving the resulting residue in water to regenerate the ammonia recovery phosphate solution. This ammonia recovery process is directed to the recovery of ammonia from coal gas or coke ovens at temperatures of 50°C. to 70°C.

U.S. Pat. Nos. 2,797,148 and 3,718,731 are directed to the recovery of ammonia from a process stream used in the production of HCN. The process of recovery uses an ammonium phosphate solution to capture the ammonia and then uses steam stripping to regenerate the ammonia from the ammonium phosphate solution. Typically, the process is operated by contacting the ammonia-containing gas with a 25% to 35% by weight ammonium phosphate solution having a pH of about 6 at a temperature of between 55°C. to 90°C. The processes in each of these patents disclose that the ammonium ion/phosphate ion ratio is at least 1.2 or greater.

U.S. Pat. No. 5,895,635 discloses a process for the recovery or regeneration of ammonia contained in the effluent from a reactor zone where ammonia, oxygen and propane/isobutane are reacted to produce acrylonitrile/methacrylonitrile. The process of recovery uses an ammonium phosphate quench solution to capture the ammonia and then regenerates the ammonium phosphate quench solution by subjecting the quench solution to elevated temperatures and pressure in order to decompose the ammonium phosphate salt. The disclosed process provides several advantages in propane ammoxidation compared to propylene ammoxidation to acrylonitrile including: (1) complete capture of by-product acrolein, thus enhancing product recovery efficiency by minimizing loss of product through, for example, reaction of acrolein with HCN in the product separation and recovery train of the process, (2) lower TOC (Total Organic Carbon) in the quench bottoms, (3) higher percentage of organics present in the quench bottoms are present as strippable/recoverable monomers instead of unrecoverable waste polymers, and (4) the ability to use a lower severity waste organic treatment (*e.g*., wet oxidation) because of the presence of lower TOC and polymers in the quench bottoms solution. An additional disclosed feature of the process is that all the waste water streams may be readily handled by conventional biotreatment processes unlike the waste streams associated with propylene ammoxidation to manufacture acrylonitrile.

Despite the aforementioned advantages, a significant drawback to ammonia recovery processes exists. For example, the operation of the process as disclosed in US 5,895,635 will lead to the formation of significant amounts of ammonium carbamate, a corrosive agent which will aggressively attack piping and equipment in the ammonia recovery process. Ammonium carbamate can also accumulate in the ammonia purification equipment, such as distillation columns, until a critical threshold concentration is achieved, at which point the carbamate is forcefully ejected from the column, creating transient pressure surges which interrupt steady-state operation and cause wide fluctuations in ammonia purity. Additionally, the presence of ammonium carbamate can lead to significant contamination of other systems, such as acrylonitrile reaction systems, which receive recycle ammonia from this process. Therefore, the industry would welcome the discovery of a process that maintains the aforementioned benefits while improving upon the prior art by minimizing the formation of ammonium carbamate in the ammonia recovery process and avoiding as much as possible process upsets and the transfer of contaminants (resulting from the presence of ammonium carbamate) to other systems, such as acrylonitrile reaction systems, which receive recycle ammonia from this process.

Accordingly, one object of the present invention is to provide an improved process for the recovery and/or regeneration of ammonia contained in the effluent from a reactor zone where an ammonia, oxygen and propane/isobutane are reacted to produce acrylonitrile/methacrylonitrile.

Another object of the present invention is to provide a process which minimizes the formation of ammonium carbamate in an ammonia recovery process.

Yet another object of the present invention is to provide a process which minimizes the accumulation of ammonium carbamate, thereby simultaneously minimizing down stream process upsets which used to result from said accumulation.

Still another object of the present invention is to provide a process which minimizes the transfer of contaminants, resulting from the presence of ammonium carbamate, to down stream processes - such as acrylonitrile reaction systems, which receive recycle ammonia from this process.

These and other objects, as well as other aspects and features and advantages of the present invention, will become apparent for those skilled in the art from consideration of the specification including the drawings and appended claims. The present invention in its various aspects is as set out in the according claims.

To achieve the foregoing objects, the present invention provides a novel process of recovering unreacted ammonia from a reactor effluent, e.g., from the effluent of a reactor wherein oxygen, ammonia and a hydrocarbon are reacted in the presence of a catalyst. This process comprises at least the following steps: (1) quenching a fluid bed reactor effluent containing unreacted ammonia with a first aqueous ammonium phosphate quench solution, thereby absorbing ammonia to form a second aqueous ammonium phosphate solution richer in ammonium ions than the first solution and (2) heating the second solution to an elevated temperature to reduce the amount of ammonium ions present to substantially the same level present in the first solution and generate a vaporous stream containing ammonia.

In one embodiment, the second aqueous ammonium phosphate solution is treated by means of a stripping gas which is substantially free of CO₂ to remove substantially all of the acrylonitrile and other useful co-products from the second solution, without increasing the CO₂ content of the second solution, prior to heating the solution to decrease the NH₄⁺ ion content.

In another embodiment, the second aqueous ammonium phosphate solution is heated in a stripping zone to remove substantially all of the acrylonitrile and other useful co-products from the second solution prior to heating the solution to decrease the NH₄⁺ ion content.

Preferably, the temperature of the first solution is between 40°C. and 80°C., and more preferably between 50°C. and 65°C.

Typically, the first quench solution has an ammonium/phosphate ratio of 1.0 or lower, preferably between 0.2 and 0.95, and more preferably between 0.6 and 0.95. The resulting pH of the first quench solution is typically between 0.9 and 3.5 The phosphate ion concentration in the first quench solution can be up to 40% by weight, preferably up to about 35% by weight.

In a still further embodiment, iron contamination of a reaction system, which receives ammonia from an ammonia recovery process, is avoided by one or more of the following techniques:
a. Physically separating iron oxide, iron containing colloidal particles, and liquid droplets from the gas stream;
b. Preventing ammonium carbamate from depositing on piping and equipment by minimizing condensation, thereby preventing corrosion of the piping; or
c. Installing piping and equipment that is not susceptible to corrosive attack by ammonium carbamate, thereby eliminating the source of iron contamination.

The present invention will be further understood by the following detailed description and reference to the accompanying drawings briefly described below:
FIG. 1 is a flow diagram of one embodiment of the present invention.
FIG. 2 is a flow diagram of another preferred embodiment of the present invention.
FIG. 3 is a plot of pH vs. N:P ratio at 60°C.
FIG. 4 is a simplified drawing of one possible apparatus which can be used for separating liquids, colloids, and particulates from a gas stream.

Ammonia reacts with carbon dioxide to yield ammonium carbamate (AC, eq. 1).

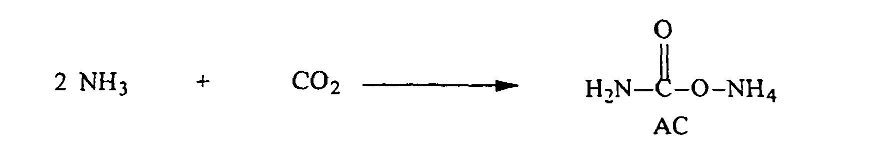

AC can dissolve into liquid that condenses on the inside wall of process piping and equipment where it can react with the iron in carbon steel to produce iron oxide (Eq. 2). Iron oxide is abrasive and particularly damaging to rotating equipment, such as ammonia compression equipment, and at high temperatures can also catalyze the decomposition of ammonia, lowering yields in processes such as acrylonitrile reaction systems. In many processes, particulate filters are installed to trap iron oxide. However, it has been discovered that, if cyanide is also present in the gas stream, iron oxide will react with cyanide to yield iron hexacyano complexes (IHC) that exist as colloidal suspensions of the corresponding ammonium salts (Eq. 3). Such colloidal suspensions are not removed by particulate filtration and pass to downstream processes where the iron hexacyano complexes can be converted back into iron oxide by reaction with oxygen in the presence of heat (Eq. 4).

(2) AC + O₂ + Carbon Steel →FeO

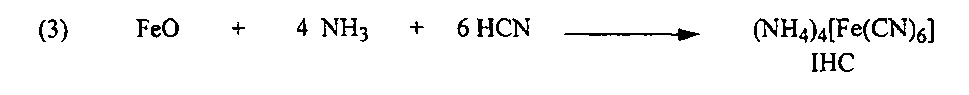

(4) (NH₄)₄[Fe(CN)₆] + O₂ → FeO

These problems are minimized by the present invention. For example, the present invention, among other things, (1) minimizes the absorption of CO₂ in recovery processes, (2) removes IHC from process streams, (3) prevents condensation on the walls of the piping and equipment which will dissolve AC from gas streams, and/or (4) uses equipment and piping that is constructed of material that is not susceptible to corrosion by AC.

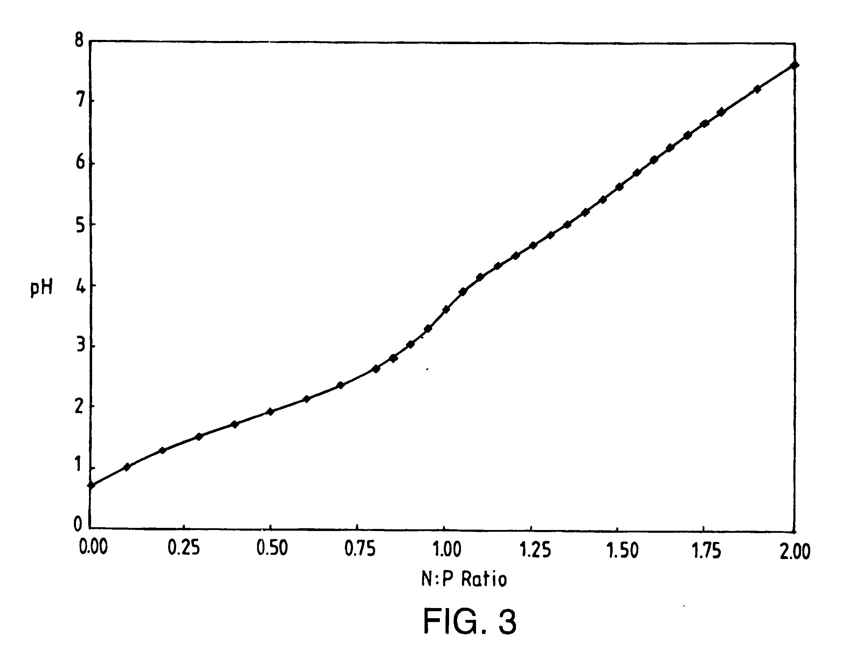

The present invention is directed to an improved process of quenching the effluent obtained from a propane ammoxidation reaction zone wherein the aforementioned problems associated with AC and its corrosion products are minimized. For example, in an ammoxidation reaction, the reaction typically takes place in a fluid bed reactor, although other types of reactors such as transport line reactors are envisioned as suitable when practicing the present invention. Fluid bed propane ammoxidation reaction conditions and fluid bed catalyst useful in propane ammoxidation are known in the art as evidenced by U.S. Pat. No. 4,746,641 herein incorporated by reference. The novel process of the present invention comprises quenching the reactor effluent obtained from the reaction of ammonia, oxygen and a hydrocarbon (*e.g*., propane and/or isobutane) in a reaction zone (*e.g.* fluid bed reactor) to produce a reaction product (*e.g*., acrylonitrile) with a first aqueous ammonium phosphate solution having a pH of about 3.5 or less, with a resulting ratio of ammonium ions to phosphate ions of not more than 1.0 (refer to Figure 3). In such a process, ammonia is absorbed to produce a second ammonium phosphate solution richer in ammonium ions than the first solution but preventing significant absorption of CO₂. Then, the second solution is heated to an elevated temperature to reduce the ammonium ion content therein to substantially the same ammonium ion content present in the first solution and generate a vaporous stream comprising ammonia, water, and CO₂. Thereafter, the molar concentration of ammonia in this vapor stream is increased. The vaporous stream containing ammonia can then be recycled to a fluid bed reactor, in a manner which minimizes iron oxide contamination of the reactor.

Preferably the pH of the first quench solution is between 1.5 and 3.3. This results in an ammonium ion/phosphate ion ratio (N:P ratio) of between about 0.3 and about 0.95. More preferably, the pH of the first quench solution is between about 1.9 and 3.0. This results in an N:P ratio of about 0.5 to 0.90.

The temperature of the first quench solution is usually between about 40°C. and about 80°C., preferably between about 50°C. and about 65°C., and more preferably between about 55°C. and about 60°C.

Given the objective of minimizing CO₂ absorption in the ammonium phosphate quench solution, one of average skill might conclude that increasing the temperature of the quench solution and perhaps lowering the operating pressure of the quench column would suffice; while it is true that such changes will reduce CO₂ absorption, they will also tend to reduce absorption of all other components in the gas stream as well, including acrolein. As it is an objective of the present invention to maintain the benefits of the prior art, such a reduction in acrolein absorption would not be desirable in the chemical production industry.

In the present invention, the utilization of a first quench solution of low pH (*e.g*., a pH ranging from about 3.5 or less) serves to block absorption of all weak acidic species. Since acrolein is not an acid compound, it is unaffected by pH changes. Because carbonic acid (the aqueous form of CO₂) has a pH of about 3.2, its tendency to absorb in the low pH solution is greatly reduced. Thus, the method of the present invention allows the temperature of the first solution to be kept low, maintaining the efficacy of acrolein absorption, while simultaneously minimizing absorption of CO₂. It should also be noted that an additional benefit of the present invention is that the absorption of HCN (pH of about 4.7), another undesirable occurrence, is also minimized when the pH of the first solution is low. Figure 3 is a graphical representation of the relationship between pH and the N:P Ratio of a phosphate solution. The data shown in Figure 3 are for a typical aqueous phosphate solution comprising 30% H₃PO₄ at 60°C and 40 psia. From Figure 3, it can be seen that the pH of the phosphate solution increases with increasing N:P ratio. For example, at an N:P ratio of 0.0, the phosphate solution will have a pH of about 0.7; at an N:P ratio of 1.0, the solution will have a pH of about 3.6; and, at an N:P ratio of 2.0, the solution will have a pH of about 7.6.

Similar data may be obtained for phosphate solutions at different H₃PO₄ concentrations, temperatures and/or pressures, as is within the ability of one of ordinary skill in the art. In general, the trend of Figure 3 (increasing pH with increasing N:P ratio) remains the same with changing H₃PO₄ concentration, although the specific pH at a given N:P ratio decreases slightly with increasing H₃PO₄ concentration. For purposes of comparison, it should be noted that an aqueous phosphate solution comprising 20% H₃PO₄ (not shown in the figure) will have a pH of about 3.9 at an N:P ratio of 1.0, while an aqueous phosphate solution comprising 40% H₃PO₄ (not shown in the figure) will have a pH of about 3.3 at an N:P ratio of 1.0.

With the benefit of this disclosure and pH vs. N:P ratio data such as that shown in Figure 3, one of ordinary skill in the art can adjust the relative concentration of ammonium ions to phosphate ions (N:P ratio) in the phosphate solution to achieve pH values in accordance with the method of the present invention.

In one embodiment of the present invention, the low pH of the first solution is maintained by purging at least a portion of the monoammonium phosphate and adding fresh, make-up phosphoric acid, in a quantity sufficient to achieve the desired pH, prior to introduction to the quench column.

In another embodiment of the present invention, the low pH of the first solution is maintained by thermally decomposing at least a portion of the monoammonium phosphate solution to generate free ammonia and phosphoric acid, in a quantity sufficient to achieve the desired pH, prior to introduction to the quench column.

In still another embodiment of the present invention, the low pH of the first solution is maintained by oxidizing at least a portion of the monoammonium phosphate in a wet oxidation process to generate oxides of nitrogen and phosphoric acid, in a quantity sufficient to achieve the desired pH, prior to introduction to the quench column.

When the aqueous monoammonium phosphate solution is utilized, the unreacted ammonia present in the reactor effluent converts the monoammonium phosphate to diammonium phosphate.During the quench procedure, the products (*e.g*., acrylonitrile, acetonitrile and/or HCN) are removed as overheads and are substantially free of ammonia. The quench solution bottom containing the diammonium phosphate also contains residual monomers (e.g. acrylonitrile) in small quantities. These monomers are, preferably, stripped and returned to the quench for further recovery and purification. Typical stripping gases for removal of the residual monomers from the quench bottoms comprise propane, nitrogen, and carbon monoxide or mixtures thereof; however, it is necessary that whatever gas is employed be substantially free of CO₂ content to avoid absorbing additional CO₂ into the solution. By substantially free it is meant that the gas contains less than 10% CO₂, preferably less than 5% CO₂, most preferably less than 1% CO₂. In a preferred embodiment, the stripping gas is a recycle stream, derived from the effluent of the acrylonitrile product purification system; CO₂ removal methods known in the art, such as adsorption or membrane separations, are used to purify the effluent such that the gas is substantially free of CO₂ before being used for stripping. (Alternatively, the residual monomers may be stripped by heating the quench bottoms so as to drive the residual monomers out of the solution without the need for a stripping gas.) The quench bottoms solution stripped of useful monomers are then regenerated at an elevated temperature and pressure to convert the diammonium phosphate back to monoammonium phosphate with the release of ammonia. The monoammonium phosphate is recovered and recycled back into the quench column. The ammonia is captured as a vapor stream which contains water and CO₂. This ammonia-rich vapor stream is heated to remove substantially all the water and the ammonia is then recycled back to the reactor. However, ammonia and CO₂ can react in the ammonia purification step as described above to form AC.

In a preferred embodiment, a caustic material is added in this purification step to convert any AC to an insoluble carbonate. Suitable caustic materials include NaOH, KOH, MgOH, CaOH and the like, as well as mixtures thereof.

In one embodiment of a typical prior art process, the stripped quench bottom containing the diammonium phosphate is passed through a wet oxidation reactor where it is treated under typical wet oxidation conditions to remove any polymers formed during the ammoxidation process. In so doing, however, the prior art process creates CO₂ within the process that will lead to the formation of ammonium carbamate. In an embodiment of the present invention wherein this prior art step is utilized, a caustic material is added to this step to convert the AC to an insoluble carbonate. Suitable caustic materials include NaOH, KOH, MgOH, CaOH and the like, as well as mixtures thereof.

In a further preferred embodiment of the present invention, the stripped quench bottom containing unrecoverable monomers and diammonium phosphate is separately treated in a phosphate decomposing unit which separates the diammonium phosphate from the residual monomers. The diammonium phosphate is then regenerated back to the monoammonium phosphate in a separate unit while the residual polymers are transferred to a wet oxidation unit for wet oxidation under conventional temperatures and pressure to produce harmless by-products such as carbon dioxide and water.

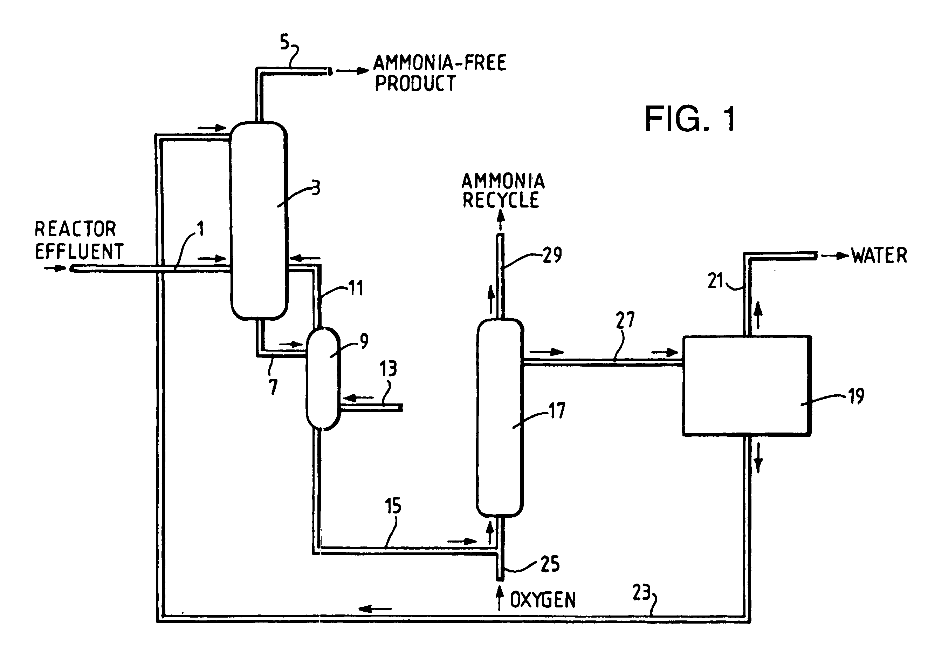

Reference will now be made to FIGS. 1 through 4, which are illustrative of some embodiments of the present invention wherein the process is applied to propane ammoxidation.

Referring to FIG. 1, reactor effluent obtained by the direct reaction of propane, ammonia and oxygen in the fluid bed reactor (not shown) over a fluid bed ammoxidation catalyst is passed via line 1 into quench column 3. In quench column 3, the reactor effluent containing product acrylonitrile and unreacted ammonia is contacted with a lean ammonium/phosphate quench solution of pH 3.5 or less which strips unreacted ammonia from the effluent without absorbing significant CO₂, producing an ammonia-free product overhead stream containing crude acrylonitrile. The crude acrylonitrile passes overhead via line 5 into conventional recovery and purification sections (not shown) for subsequent recovery of commercially pure acrylonitrile, crude acetonitrile and hydrogen cyanide. Examples of conventional recovery and purification procedures can be found in U.S. Pat. No. 3,936,360 incorporated by reference herein. The quench bottoms leave quench column 3 via line 7 and enter a quench stripper 9. A stripping gas, substantially free of CO₂, comprising a recycle stream comprising a mixture of propane, carbon monoxide, and nitrogen is passed via line 13 into stripper 9 to remove any residual volatile impurities, such as, for example, acrylonitrile, acetonitrile or hydrogen cyanide contained in the quench bottoms. (Volatile is used in a comparative sense as to the ammonia chemically bound in the ammonium phosphate solution.) Alternatively, the quench bottoms may be fed to stripper 9 where they are heated so as to drive off any of the residual volatile impurities albeit at a temperature lower than that used to cause decomposition of the diammonium phosphate present in the quench bottoms. The overhead stripper gas 13 containing these residual monomers is recycled back into quench column 3 via line 11 for further recovery of useful products. The stripped quench bottoms are passed from stripper 9 via line 15 into a wet oxidation reactor 17 wherein oxygen is passed via line 25 and a conventional catalytic wet oxidation take place to remove unwanted impurities such as polymers. In addition, the diammonium phosphate contained in the quench stripper bottoms is heated to free the ammonia and convert the diammonium phosphate in solution to monoammonium phosphate. An optional caustic material is added to wet oxidation reactor 17 to convert ammonium carbamate to an insoluble carbonate. Suitable caustic materials include NaOH, KOH, MgOH, CaOH and the like, as well as mixtures thereof.

The monoammonium phosphate solution is passed from reactor 17 via line 27 into evaporator 19 where excess water is removed from the solution. This excess water is passed from evaporator 19 via line 21 for recycle or disposal. The concentrated weak monoammonium phosphate solution is passed from evaporator 19 via line 23 for recycle into quench column 3. The ammonia released during the heat treatment in wet oxidation reactor 17 is passed from reactor 17 via line 29 for recycle directly into the fluid bed reactor (not shown). Any CO₂ produced in the wet oxidation process can react with the ammonia according to reaction 1 to form AC. If condensation occurs on the inside wall of line 29, dissolved AC can corrosively attack the piping material. In one embodiment of the present invention, the temperature of line 29 is maintained high enough to prevent condensation on the inside of the line. The temperature of the line may be maintained by heating the line with steam or electrical tracing or by jacketing. Insulation may also be present. In one embodiment of the present invention, the temperature of the line is maintained above the condensation temperature of the gas and below about 350°C. More preferably, the temperature of the line is maintained in the range from about 70°C to about 200°C.

In still another embodiment of the present invention, line 29 and reactor 17 are constructed of a material that is not susceptible to corrosion by AC. In one embodiment of the present invention, line 29 and reactor 17 are constructed from a metal that has a lower iron content than carbon steel. Preferred material include stainless steel, L series stainless steel, Duplex 2205, Hastelloys, Inconels, and Zirconium. In one embodiment of the present invention, line 29 and reactor 17 are constructed from Type 316L stainless steel. In an alternative embodiment of the present invention, the inside wall of line 29and reactor 17 are lined with a non-metallic such as Teflon or glass. It is further contemplated that in some situations it may be advantageous to construct line 29 and reactor 17 from different corrosion-resistant materials, and further that equipment, such as the reactor itself, may employ more than one material of construction - for example, the reactor wall may be lined/clad with a non-metallic material such as glass or resin and the internals of the reactor may be of corrosion resistant metal.

Typical wet oxidation conditions are utilized for the destruction of the unwanted polymers obtained during the process. Typical catalysts for wet oxidation are soluble salts of copper and iron, oxides of copper, zinc, manganese and cerium and noble metals and are well known in the prior art. See, for example, Ind. Eng. Chem. Res., 1995 Vol. 34, Pages 2-48, incorporated by reference herein. The wet oxidation reaction is designed for normal operation. Typically, wet oxidation is run at a pressure of between about 600 to 3000 psia and a temperature of 200°C. to 650°C.

In an alternate embodiment, the ammonia containing gas in line 29 is treated to provide a gas with reduced ammonium carbamate (AC) concentration through one or more of the following methods:
- contacting the gas with a scrubbing solution to convert AC to carbonate, wherein the scrubbing solution comprises a caustic material (suitable caustic materials are as previously described)
- contacting the gas with a basic Ion Exchange Resin to remove AC
- contacting the gas with an adsorbent to remove free CO2 and/or AC
- condensing the gas, contacting the gas with sacrificial iron, such as a carbon steel mesh, to react away the AC, and then heating the condensate sufficiently to revaporize the ammonia
- condensing the gas, contacting the condensate with caustic material to convert AC to carbonate (suitable caustic materials are as previously described), and then heating the condensate sufficiently to revaporize the ammonia
- condensing the gas, contacting the condensate with an electrode to electrolytically decompose the AC, and then heating the condensate sufficiently to revaporize the ammonia
- subjecting the gas to elevated temperature and pressure sufficient to convert the AC to urea, and then removing the urea.

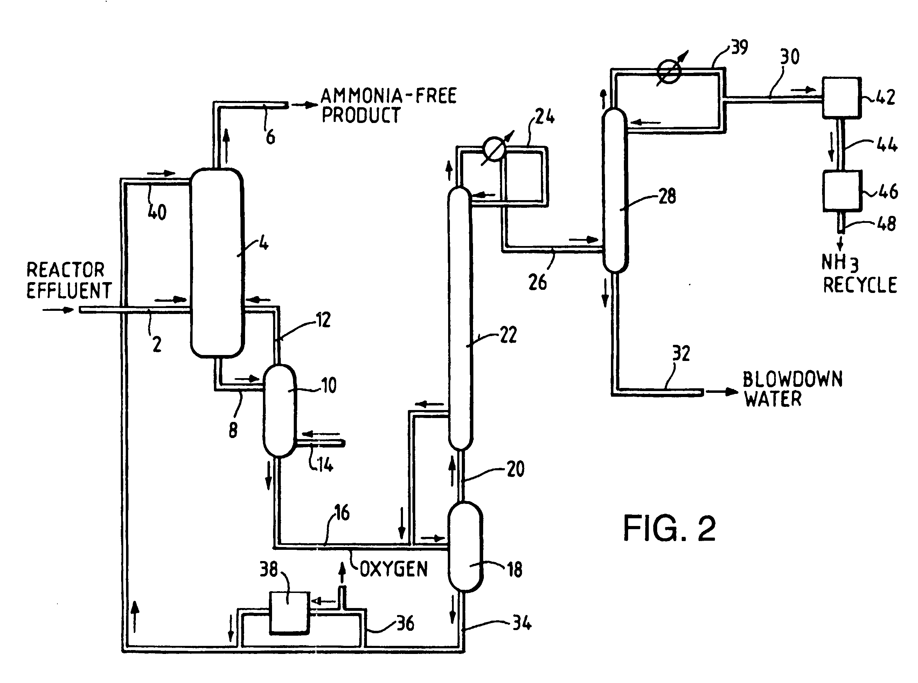

With reference to FIG. 2 a further preferred embodiment of the present invention is described. The process illustrated in FIG. 2 is substantially the same as that of FIG. 1 except that the phosphate decomposition takes place in a separate unit followed by wet oxidation in a different unit. The reactor effluent obtained by the direct ammoxidation of propane, oxygen and ammonia in a fluid bed reactor (not shown) is passed from the fluid bed reactor via line 2 into quench 4. The reactor effluent containing crude acrylonitrile and unreacted ammonia is contacted in quench 4 with an aqueous monoammonium phosphate solution of pH 3.5 or less which enters quench 4 via line 40. The phosphate solution removes the unreacted ammonia from the reactor effluent without absorbing significant CO₂, allowing the ammonia-free products (crude acrylonitrile) to pass overhead from quench 4 via line 6. The crude acrylonitrile passing overhead via line 6 is directed to a conventional recovery and purification section for recovery of commercially pure acrylonitrile, crude acetonitrile and HCN. Quench bottoms are passed from quench 4 via line 8 into quench stripper 10 where a stripping gas (having the same composition as described above) enters the lower portion of the bottom stripper 10 via line 14 and passes upward through the quench bottoms to strip the quench bottoms of any useful monomers present in the bottoms such as acrylonitrile, acetonitrile and hydrogen cyanide. The stripper gas containing useful monomers is then passed from stripper 10 overhead via line 12 into quench 4 for further recovery and purification. As in the previous embodiment, these useful monomers can also be recovered by merely heating the quench bottoms in quench stripper 10. The stripped quench bottoms move from stripper 10 via line 16 to phosphate decomposer 18. In phosphate decomposer 18, the diammonium phosphate present in the stripped quench bottom is converted to free ammonium and monoammonium phosphate by heating to an elevated temperature (100°C. to 300°C.). Typically, the pressure is between 1 to 5 atmospheres (atmospheric to 75 psia). Oxygen may be present but is not required. The resulting monoammonium phosphate solution is passed from decomposer 18 via line 34 for recycle via line 40 into quench 4. The free ammonia generated during phosphate conversion in reactor 18 is passed via overhead line 20 into an ammonia rectification unit 22 wherein the free ammonia is purified and passed on to ammonia stripper 28 via line 26 to recover the ammonia for recycle into the reactor (not shown) for manufacture of acrylonitrile or may be recycled via line 24 to rectification unit 22 prior to going to ammonia stripper 28. Water is recovered from ammonia stripper unit 28 and passed via line 32 for recycle or disposal. Caustic material (not shown) is added to ammonia stripper 28 directly, or optionally, indirectly via lines 26 or 39, to convert AC to insoluble carbonate. Caustic material may optionally be added to rectification unit 22 as well. Suitable caustic materials include NaOH, KOH, MgOH, CaOH and the like, as well as mixtures thereof.

Because Ammonia and CO₂ are present in this part of the process, AC will form according to reaction 1, above, and Lines 24, 26, 39, and 30, as well as stripper 28, its condenser, and the condenser on column 22 (here forward known as the "NH₃ purification equipment") will be exposed to AC.

If condensation occurs on the inside wall(s) of the NH₃ purification equipment, the dissolved AC can corrosively attack this equipment. In one embodiment of the present invention, the temperature of line 30 is maintained high enough to prevent condensation on the inside of the line. The temperature of the "NH₃ purification equipment" may be maintained by heating with steam or electrical tracing or by jacketing. Insulation may also be present. In one embodiment of the present invention, the temperature of the "NH₃ purification equipment" is maintained above the condensation temperature of the gas and below about 350°C. More preferably, the temperature of the line is maintained in the range from about 70°C to about 200°C.

In still another embodiment of the present invention, the "NH₃ purification equipment" is constructed of a material that is not susceptible to corrosion by AC. In one embodiment of the present invention, the "NH₃ purification equipment" is constructed from a metal that has a lower iron content than carbon steel. Preferred material include stainless steel, L series stainless steel, Duplex 2205, Hastelloys, Inconels, and Zirconium. In one embodiment of the present invention, the "NH₃ purification equipment" is constructed from Type 316L stainless steel. In an alternative embodiment of the present invention, the inside wall(s) of the "NH3 purification equipment" are lined with a non-metallic such as Teflon or glass. It is further contemplated that in some situations it may be advantageous to construct different components of the "NH₃ purification equipment" from different corrosion-resistant materials, and further that equipment, such as columns 22 and 28 and their respective condensers, may employ more than one material of construction - for example, in the case of the condenser of stripper 28, the condenser tubesheet may be lined/clad with a non-metallic material such as glass or resin and the tubes may be of corrosion-resistant metal.

The NH₃ is passed from stripper 28 via line 30 for recycle or is passed via line 39 to stripper 28 for processing prior to entry into line 30 for recycle. In one embodiment of the present invention, gas exiting via line 30 is transferred to optional compressor 42. In one embodiment of the present invention, compressor 42 is constructed from materials that are resistant to corrosion by AC. Suitable materials are as listed above. In an especially preferred embodiment, the compressor is operated at an elevated temperature, such that the gas is discharged at a temperature between about 80°C and 350°C. In an alternative embodiment of the present invention, optional compressor 42 is absent and lines 30 and 44 are contiguous.

In one aspect of the present invention, lines 30 and 44 are constructed of a material that is not susceptible to corrosion by AC. Suitable material are as listed above. In one embodiment of the present invention, lines 30 and 44 are constructed from 316L stainless. In an alternative embodiment of the present invention, the inside walls of lines 30 and 44 are lined with a non-metal material, preferably Teflon or glass.

In another embodiment of the present invention, the temperature of the gas inside lines 30 and 44 is maintained high enough to prevent condensation in these lines or in related equipment. In one embodiment of the present invention, lines 30 and 44 as well as any intervening equipment are heated with steam or electrical tracing to prevent condensation on the inside of the lines. Alternatively, lines 30 and 44 and any intervening equipment are heated with jacketing. Insulation may also be present. In these embodiments, the lines and equipment are maintained above the condensation temperature of the gas and below about 350°C, more preferably between 70°C and 200°C.

In another embodiment of the present invention, the gas in lines 30 and 44 is passed through at least one heat exchanger to elevate and maintain the temperature of the gas above its condensation temperature and below about 350°C. More preferably, the temperature of the gas is maintained in the range from about 70°C to about 200°C

Condensation can also be minimized by operating ammonia stripper column 28 and its condenser such that the concentration of water in the purified gas stream entering line 30 is minimized. The concentration of ammonia in the gas stream entering line 30 is preferably greater than 75%, more preferably greater than 90%, and even more preferably greater than 95%.

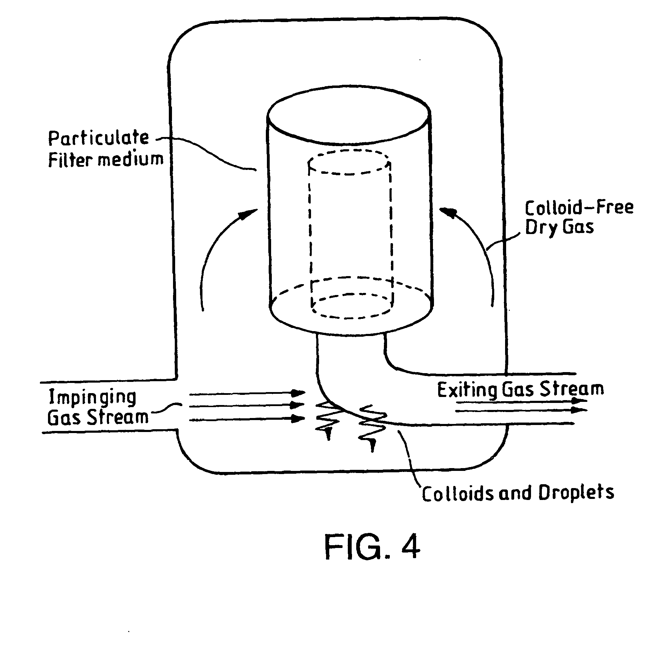

In another embodiment of the present invention, gas in line 44 passes through a zone 46 where impurities are removed from the gas stream. The zone comprises a first component that separates colloidal particles and liquid droplets from the gas stream and a second component that separates particulate matter from the gas stream. In one embodiment of the present invention, the two components are combined into one apparatus. Referring to Figure 4, in one embodiment of the present invention, the gas in line 44 is directed into a chamber, wherein a vector change in the gas stream causes the colloidal material and liquid droplets entrained in the gas to impact internal structures, such as baffles, impingement plates, and (as shown here) the piping elbow, as well as the sides of the chamber. The colloid- and liquid-free gas then passes through particulate filtering media which is off the line of the impinging gas stream before exiting the chamber. In an alternative embodiment of the present invention, the zone in which impurities are separated from the gas stream may comprise one or more cyclones or impingement separators to physically remove droplets and colloidal materials from the gas stream followed by one or more filters to remove particulate from the gas stream.

In an alternate embodiment, the ammonia containing gas in line 30 is treated to provide a gas with reduced ammonium carbamate (AC) concentration through one or more of the following methods:
- contacting the gas with a scrubbing solution to convert AC to carbonate, wherein the scrubbing solution comprises a caustic material (suitable caustic materials are as previously described)
- contacting the gas with a basic Ion Exchange Resin to remove AC
- contacting the gas with an adsorbent to remove free CO2 and/or AC
- condensing the gas, contacting the gas with sacrificial iron, such as a carbon steel mesh, to react away the AC, and then heating the condensate sufficiently to revaporize the ammonia
- condensing the gas, contacting the condensate with caustic material to convert AC to carbonate (suitable caustic materials are as previously described), and then heating the condensate sufficiently to revaporize the ammonia
- condensing the gas, contacting the condensate with an electrode to electrolytically decompose the AC, and then heating the condensate sufficiently to revaporize the ammonia
- subjecting the gas to elevated temperature and pressure sufficient to convert the AC to urea, and then removing the urea.

At least a portion of the weak monoammonium phosphate solution passed from decomposer 18 via line 34 may be sent through wet oxidation unit 38 via line 36 for removal of polymers and conversion of these unwanted materials into harmless by-products such as hydrogen, carbon monoxide and carbon dioxide.

As described previously, the wet oxidation may be performed under conventional conditions known in the art.

While the invention has been described in conjunction with specific embodiments herein, it is evident that many alternatives, modifications and variations will be apparent to those skilled in the art in light of the foregoing description. Accordingly it is intended to embrace all such alternatives and modifications in variations as for within the spirit and broad scope of the appended claims.

## Claims

1. A process for the recovery of ammonia from a reactor effluent stream comprising:
contacting a gaseous reactor effluent stream containing ammonia with a first aqueous ammonium phosphate solution having a pH of 3.5 or less, in a quench zone, to absorb substantially all of the ammonia present in the reactor effluent stream to form a second aqueous ammonium phosphate solution richer in ammonium ions than said first aqueous ammonium phosphate solution;
contacting said second aqueous ammonium phosphate solution with a stripping gas, which is substantially free of carbon dioxide, to remove volatile impurities contained in said second aqueous ammonium phosphate solution; and
heating said stripped second aqueous ammonium phosphate solution to an elevated temperature sufficient to reduce the amount of ammonium ions in said stripped second aqueous ammonium phosphate solution back to substantially the same level present in said first aqueous ammonium phosphate solution to thereby generate a vapor stream comprising ammonia and an aqueous stream.

2. The process of claim 1, wherein the heating of said stripped second aqueous ammonium phosphate solution to an elevated temperature takes place in a wet oxidation reactor at wet oxidation conditions to simultaneously remove unwanted impurities from said stripped second aqueous ammonium phosphate solution and reduce the ammonium ion concentration to substantially the same level present in said first aqueous ammonium phosphate solution, a caustic material being added to said wet oxidation reactor to convert any ammonium carbamate formed to an insoluble carbonate.

3. The process of claim 1, wherein the heating of said stripped second aqueous ammonium phosphate solution to an elevated temperature takes place in a wet oxidation reactor at wet oxidation conditions to simultaneously remove unwanted impurities from said stripped second aqueous ammonium phosphate solution and reduce the ammonium ion concentration to substantially the same level present in said first aqueous ammonium phosphate solution, and wherein said vapor stream comprising ammonia is recycled to the reactor through a line, the interior wall of which is maintained at temperature above the condensation temperature of the vapor stream.

4. The process of claim 1, wherein the heating of said stripped second aqueous ammonium phosphate solution to an elevated temperature takes place in a wet oxidation reactor at wet oxidation conditions to simultaneously remove unwanted impurities from said stripped second aqueous ammonium phosphate solution and reduce the ammonium ion concentration to substantially the same level present in said first aqueous ammonium phosphate solution, and wherein said vapor stream comprising ammonia is recycled to the reactor through a line, said wet oxidation reactor and said line being constructed of a material that is not susceptible to corrosion by ammonium carbamate.

5. The process of claim 1, wherein the heating of said stripped second aqueous ammonium phosphate solution to an elevated temperature takes place in a wet oxidation reactor at wet oxidation conditions to simultaneously remove unwanted impurities from said stripped second aqueous ammonium phosphate solution and reduce the ammonium ion concentration to substantially the same level present in said first aqueous ammonium phosphate solution, and wherein the so-generated vapor stream comprising ammonia is treated to reduce the concentration of any ammonium carbamate therein.

6. The process of claim 1, wherein said aqueous stream having reduced ammonium ion concentration is recycled to said quench zone.

7. The process of claim 6, wherein at least a portion of said aqueous stream is subjected to a wet oxidation reaction at wet oxidation conditions to remove unwanted impurities from said aqueous stream prior to recycle to said quench zone

8. The process of claim 1, wherein said vapor stream comprising ammonia is recycled to said reactor, said vapor stream having been contacted with caustic material to convert any ammonium carbamate to a carbonate.

9. The process of claim 1, wherein said vapor stream comprising ammonia is recycled to said reactor through ammonia purification equipment and wherein the temperature of the ammonia purification equipment is maintained at a temperature above the condensation temperature of the vapor stream.

10. The process of claim 1, wherein said vapor stream comprising ammonia is recycled to said reactor through ammonia purification equipment and wherein the ammonia purification equipment is constructed of a material that is not susceptible to corrosion by ammonium carbamate.

## Patentansprüche

1. Verfahren zur Rückgewinnung von Ammoniak aus einem Reaktorabflußstrom, umfassend:
das Inkontaktbringen eines gasförmigen Reaktorabflußstroms, enthaltend Ammoniak, mit einer ersten wäßrigen Ammoniumphosphatlösung mit einem pH von 3,5 oder weniger in einer Quenchzone, um im wesentlichen das sämtliche Ammoniak, das in dem Reaktorabflußstrom vorliegt, unter Bildung einer zweiten wäßrigen Ammoniumphosphatlösung, die reicher an Ammoniumionen als die erste wäßrige Ammoniumphosphatlösung ist, zu absorbieren;
das Inkontaktbringen der zweiten wäßrigen Ammoniumphosphatlösung mit einem Abstreifgas, welches im wesentlichen frei von Kohlendioxid ist, um flüchtige Verunreinigungen, die in der zweiten wäßrigen Ammoniumphosphatlösung enthalten sind, zu entfernen, und
das Erwärmen der abgestreiften zweiten wäßrigen Ammoniumphosphatlösung auf eine erhöhte Temperatur, die ausreichend ist, die Menge an Ammoniumionen in der abgestreiften zweiten wäßrigen Ammoniumphosphatlösung zurück auf im wesentlichen den gleichen Grad, der in der ersten wäßrigen Ammoniumphosphatlösung vorliegt, zu reduzieren, um **dadurch** einen Dampfstrom, umfassend Ammoniak, und einen wäßrigen Strom zu erzeugen.

2. Verfahren gemäß Anspruch 1, wobei das Erwärmen der abgestreiften zweiten wäßrigen Ammoniumphosphatlösung auf eine erhöhte Temperatur in einem Naßoxidationsreaktor bei Naßoxidationsbedingungen stattfindet, um gleichzeitig ungewollte Verunreinigungen von der abgestreiften zweiten wäßrigen Ammöniumphosphatlösung zu entfemen und die Ammoniumionenkonzentration auf im wesentlichen den gleichen Grad, vorliegend in der ersten wäßrigen Ammoniumphosphatlösung, zu reduzieren, wobei ein kaustisches Material zu dem Naßoxidationsreaktor gegeben wird, um jegliches gebildete Ammoniumcarbamat in ein unlösliches Carbonat umzuwandeln.

3. Verfahren gemäß Anspruch 1, wobei das Erwärmen der abgestreiften zweiten wäßrigen Ammoniumphosphatlösung auf eine erhöhte Temperatur in einem Naßoxidationsreaktor bei Naßoxidationsbedingungen stattfindet, um gleich-zeitig ungewollte Verunreinigungen von der abgestreiften zweiten wäßrigen Ammoniumphosphatlösung zu entfernen und die Ammoniumionenkonzentration auf im wesentlichen den gleichen Grad, vorliegend in der ersten wäßrigen Ammoniumphosphatlösung, zu reduzieren, und wobei der Dampfstrom, umfassend Ammoniak, zu dem Reaktor über eine Leitung rückgeführt wird, wobei dessen innere Wand bei einer Temperatur von oberhalb der Kondensationstemperatur des Dampfstroms gehalten wird.

4. Verfahren gemäß Anspruch 1, wobei das Erwärmen der abgestreiften zweiten wäßrigen Ammoniumphosphatlösung auf eine erhöhte Temperatur in einem Naßoxidationsreaktor bei Naßoxidationsbedingungen stattfindet, um gleichzeitig ungewollte Verunreinigungen von der abgestreiften zweiten wäßrigen Ammoniumphosphatlösung zu entfernen, und die Ammoniumionenkonzentration auf im wesentlichen den gleichen Grad, vorliegend in der ersten wäßrigen Ammoniumphosphatlösung, zu reduzieren, und wobei der Dampfstrom, umfassend Ammoniak, zu dem Reaktor über eine Leitung rückgeführt wird, wobei der Naßoxidationsreaktor und die Leitung aus einem Material aufgebaut sind, das keiner Korrosion durch Ammoniumcarbamat unterliegt.

5. Verfahren gemäß Anspruch 1, wobei das Erwärmen der abgestreiften zweiten wäßrigen Ammoniumphosphatlösung auf eine erhöhte Temperatur in einem Naßoxidationsreaktor bei Naßoxidationsbedingungen stattfindet, um gleichzeitig ungewollte Verunreinigungen von der abgestreiften zweiten wäßrigen Ammoniumphosphatlösung zu entfernen, und die Ammoniumionenkonzentration auf im wesentlichen den gleichen Grad, vorliegend in der ersten wäßrigen Ammoniumlösung, zu reduzieren, und wobei der derart erzeugte Dampfstrom, umfassend Ammoniak, behandelt wird, um die Konzentration von jeglichem Ammoniumcarbamat darin zu reduzieren.

6. Verfahren gemäß Anspruch 1, wobei der wäßrige Strom mit verminderter Ammoniumionenkonzentration zu der Quenchzone rückgeführt wird.

7. Verfahren gemäß Anspruch 6, wobei mindestens ein Teil des wäßrigen Stroms einer Naßoxidationsreaktion bei Naßoxidationsbedingungen unterworfen wird, um ungewollte Verunreinigungen von dem wäßrigen Strom vor der Rückführung zu der Quenchzone zu entfernen.

8. Verfahren gemäß Anspruch 1, wobei der Dampfstrom, umfassend Ammoniak, zu dem Reaktor rückgeführt wird, wobei der Dampfstrom mit kaustischem Material in Kontakt gebracht worden ist, um jegliches Ammoniumcarbamat zu einem Carbonat umzuwandeln.

9. Verfahren gemäß Anspruch 1, wobei der Dampfstrom, umfassend Ammoniak, zu dem Reaktor durch eine Ammoniakreinigungsausstattung rückgeführt wird, und wobei die Temperatur der Ammoniakreinigungsausstattung bei einer Temperatur von oberhalb der Kondensationstemperatur des Dampfstroms gehalten wird.

10. Verfahren gemäß Anspruch 1, wobei der Dampfstrom, umfassend Ammoniak, zu dem Reaktor durch eine Ammoniakreinigungsausstattung rückgeführt wird, und wobei die Ammoniakreinigungsausstattung aus einem Material aufgebaut ist, das keiner Korrosion durch Ammoniumcarbamat unterliegt.

## Revendications

1. Procédé de récupération d'ammoniac à partir d'un courant d'effluents d'un réacteur, comprenant:
la mise en contact d'un courant d'effluents gazeux du réacteur contenant de l'ammoniac avec une première solution aqueuse de phosphate d'ammonium ayant un pH d'au plus 3,5, dans une zone de désactivation, pour absorber pratiquement tout l'ammoniac présent dans le courant d'effluents du réacteur pour former une deuxième solution aqueuse de phosphate d'ammonium plus riche en ions ammonium que ladite première solution aqueuse de phosphate d'ammonium;
la mise en contact de ladite deuxième solution aqueuse de phosphate d'ammonium avec un gaz de lavage qui est essentiellement dépourvu de dioxyde de carbone, pour séparer les impuretés volatiles contenues dans ladite deuxième solution aqueuse de phosphate d'ammonium; et
le chauffage de ladite deuxième solution aqueuse de phosphate d'ammonium lavée à une température élevée suffisante pour ramener la quantité d'ions ammonium dans ladite deuxième solution aqueuse de phosphate d'ammonium lavée pratiquement au même niveau que celui présent dans ladite première solution aqueuse de phosphate d'ammonium de manière à produire un courant de vapeurs comprenant de l'ammoniac et un courant aqueux.

2. Procédé selon la revendication 1, dans lequel on effectue le chauffage de ladite deuxième solution aqueuse de phosphate d'ammonium lavée à une température élevée dans un réacteur d'oxydation à l'état humide dans des conditions d'oxydation à l'état humide pour en même temps séparer les impuretés indésirables de ladite deuxième solution aqueuse de phosphate d'ammonium lavée et réduire la concentration d'ions ammonium pratiquement au même niveau que celui présent dans ladite première solution aqueuse de phosphate d'ammonium, en ajoutant une substance caustique dans ledit réacteur d'oxydation à l'état humide pour convertir tout carbamate d'ammonium formé en un carbonate insoluble.

3. Procédé selon la revendication 1, dans lequel on effectue le chauffage de ladite deuxième solution aqueuse de phosphate d'ammonium lavée à une température élevée dans un réacteur d'oxydation à l'état humide dans des conditions d'oxydation à l'état humide pour en même temps séparer les impuretés indésirables de ladite deuxième solution aqueuse de phosphate d'ammonium lavée et réduire la concentration d'ions ammonium pratiquement au même niveau que celui présent dans ladite première solution aqueuse de phosphate d'ammonium, et dans lequel ledit courant de vapeurs comprenant de l'ammoniac est recyclé dans le réacteur par une conduite dont la paroi interne est maintenue à une température supérieure à la température de condensation du courant de vapeurs.

4. Procédé selon la revendication 1, dans lequel on effectue le chauffage de ladite deuxième solution aqueuse de phosphate d'ammonium lavée à une température élevée dans un réacteur d'oxydation à l'état humide dans des conditions d'oxydation à l'état humide pour en même temps séparer les impuretés indésirables de ladite deuxième solution aqueuse de phosphate d'ammonium lavée et réduire la concentration d'ions ammonium pratiquement au même niveau que celui présent dans ladite première solution aqueuse de phosphate d'ammonium, et dans lequel ledit courant de vapeurs comprenant de l'ammoniac est recyclé dans le réacteur par une conduite, ledit réacteur d'oxydation à l'état humide et ladite conduite étant construits en un matériau qui n'est pas sensible à la corrosion par le carbamate d'ammonium.

5. Procédé selon la revendication 1, dans lequel on effectue le chauffage de ladite deuxième solution aqueuse de phosphate d'ammonium lavée à une température élevée dans un réacteur d'oxydation à l'état humide dans des conditions d'oxydation à l'état humide pour en même temps séparer les impuretés indésirables de ladite deuxième solution aqueuse de phosphate d'ammonium lavée et réduire la concentration d'ions ammonium pratiquement au même niveau que celui présent dans ladite première solution aqueuse de phosphate d'ammonium, et dans lequel on traite le courant de vapeurs comprenant de l'ammoniac ainsi produit pour en réduire la concentration en carbamate d'ammonium.

6. Procédé selon la revendication 1, dans lequel ledit courant aqueux ayant une concentration en ions ammonium réduite est recyclé dans ladite zone de désactivation.

7. Procédé selon la revendication 6, dans lequel on soumet au moins une portion dudit courant aqueux à une réaction d'oxydation à l'état humide dans des conditions d'oxydation à l'état humide pour séparer les impuretés indésirables dudit courant aqueux avant de le recycler dans ladite zone de désactivation.

8. Procédé selon la revendication 1, dans lequel ledit courant de vapeurs comprenant de l'ammoniac est recyclé dans ledit réacteur, ledit courant de vapeurs ayant été mis en contact avec une substance caustique pour la conversion de tout carbamate d'ammonium en un carbonate.

9. Procédé selon la revendication 1, dans lequel ledit courant de vapeurs comprenant de l'ammoniac est recyclé dans ledit réacteur par l'intermédiaire d'un équipement de purification d'ammoniac et dans lequel la température dudit équipement de purification d'ammoniac est maintenue à une température supérieure à la température de condensation du courant de vapeurs.

10. Procédé selon la revendication 1, dans lequel ledit courant de vapeurs comprenant de l'ammoniac est recyclé dans ledit réacteur par l'intermédiaire d'un équipement de purification d'ammoniac et dans lequel l'équipement de purification d'ammoniac est construit en un matériau qui n'est pas sensible à la corrosion par le carbamate d'ammonium.
